**Europäisches Patentamt**

**European Patent Office**

**Office européen des brevets**

(19)

(11) Veröffentlichungsnummer : **0 076 354 B2**

(12) # NEUE EUROPÄISCHE PATENTSCHRIFT

(45) Veröffentlichungstag der neuen Patentschrift :
**04.11.92 Patentblatt 92/45**

(21) Anmeldenummer : **82100942.0**

(22) Anmeldetag : **10.02.82**

(51) Int. Cl.⁵ : **A61F 13/02**, A61N 2/08

(54) **Magnetpflaster.**

(30) Priorität : **02.10.81 DE 3139280**
**03.12.81 DE 3147852**

(43) Veröffentlichungstag der Anmeldung :
**13.04.83 Patentblatt 83/15**

(45) Bekanntmachung des Hinweises auf die
Patenterteilung :
**27.08.86 Patentblatt 86/35**

(45) Bekanntmachung des Hinweises auf die
Entscheidung über den Einspruch :
**04.11.92 Patentblatt 92/45**

(84) Benannte Vertragsstaaten :
**AT BE CH DE FR GB IT LI LU NL SE**

(56) Entgegenhaltungen :
**DE-A- 2 806 088**

(56) Entgegenhaltungen :
**DE-A- 2 914 904**
**DE-A- 3 041 065**
**DE-U- 1 885 256**
**DE-U- 6 904 160**
**FR-A- 1 215 110**
**US-A- 3 426 754**
**B. & H. HELWIG: "Moderne Arzneimittel". 5.**
**Auflage, 1980, Wissenschaftlicher Verlag,**
**Stuttgart**

(73) Patentinhaber : **Latzke, Arno Walter**
**Mühltobel 946**
**Ch-9429 Zelg/Wolfhalden (CH)**

(72) Erfinder : **Latzke, Arno Walter**
**Mühltobel 946**
**Ch-9429 Zelg/Wolfhalden (CH)**

(74) Vertreter : **Werner, Hans-Karsten, Dr. et al**
**Deichmannhaus am Hauptbahnhof**
**W-5000 Köln 1 (DE)**

EP 0 076 354 B2

## Beschreibung

Gegenstand der Erfindung sind Magnetpflaster zu Heilzwecken, insbesondere zur magnetischen Therapie von Rheuma, Gelenkschmerzen, Ischias Hexenschuss und sonstigen Erkrankungen, die mit magnetischen Feldern erfolgreich behandelt werden können.

Es ist bekannt, die magnetische Heilung mit aufwendigen Apparaturen oder auch durch magnetische Heilpflaster bestehend aus einem Ferritplättchen und einem selbstklebenden Heftpflaster zu behandeln. Derartige magnetische Heilpflaster sind beispielsweise aus dem deutschen Gebrauchsmuster 7 919 808 bekannt. Der Nachteil dieser bekannten magnetischen Heilpflaster besteht darin, dass sie ihre Wirkung nur dann entfalten, wenn sie an den richtigen Stellen aufgeklebt werden. Das Auffinden der optimalen Befestigungspunkte ist insbesondere für den Laien, aber auch für den weniger routinierten Heilpraktiker oder Arzt mit Schwierigkeiten verbunden. Es wurde jetzt gefunden, dass die Erfolgsquote mit den bisher bekannten magnetischen Heilpflastern noch gesteigert werden könnte, wenn die Ferritplättchen jeweils an der optimalen Stelle befestigt würden.

Aus dem deutschen Gebrauchsmuster 6 904 160 ist eine Vorrichtung zur Erzeugung eines magnetischen Feldes an Körperteilen bekannt, bei denen der Magnetträger ein magnetisierbarer, elastischer, flacher Körper, vorzugsweise in Folienform ist. Diese Vorrichtung soll dadurch am Körper befestigt werden, dass die Befestigungselemente wie Haft- und Klebestreifen, Schlaufen, Bänder, Bügel und dergleichen vorhanden sind. Eine selbstklebende Schicht ist diesem Stand der Technik nicht zu entnehmen. Die zweite ebenfalls beanspruchte Ausführungsform, nämlich die Befestigung mit Haft- und Klebestreifen ist nur beiläufig erwähnt.

Als Magnetträger werden in dem Gebrauchsmuster 6 904 160 magnetisierbare, elastische flache Körper, vorzugsweise in Folienform genannt, welche entweder aus einer einseitig oder beiderseitig mit magnetischem Material beschichteten nicht magnetisierten Folie bestehen oder aus in eine Bindesubstanz eingebetteten magnetisierbaren Partikeln und/oder Einzelmagneten. Nach der bevorzugten Ausführungsform sollen diese magnetisierbaren Schichten axial magnetisiert sein, wobei die Stärke der Magnetisierung von Punkt zu Punkt unterschiedlich sein kann. Unter einer axialen Magnetisierung versteht man eine Magnetisierung senkrecht zur Folienfläche oder in Richtung ihrer geringsten Dicke; man spricht auch von einer Durchmagnetisierung. Hierbei entsteht auf der einen Oberfläche der Folie ein Nordpol bzw. Pluspol und auf der entgegengesetzten Fläche der Folie ein Südpol bzw. Minuspol. Derartige axial magnetisierte Folien weisen stets an den Kanten etwas höhere Feldstärken auf, wodurch der Eindruck entsteht, dass sie von Punkt zu Punkt unterschiedlich stark magnetisiert seien. Eine axiale Magnetisierung ist bei gleicher Schichtdicke stets um ca. den Faktor 10 schwächer als eine abwechselnd positive und negative Magnetisierung. Die nach diesem Stand der Technik erzielbaren Feldstärken sind somit relativ schwach und therapeutisch kaum wirksam.

Aus der FR-A-1 215 110, entsprechend der DE-U-1 885 256 ist ein therapeutisches Gerät zur Blutkreislaufregulierung bekannt welches aus einem um den menschlichen Körper oder an ein Glied anlegbares Band besteht mit mehreren daran befestigten Dauermagneten. Insbesondere handelt es sich hierbei also um Armbänder, Uhrenarmbänder und Gürtel, von denen behauptet wird, dass sie ihre Wirksamkeit praktisch im ganzen Körper entwickeln. Tatsächlich lässt sich jedoch die therapeutische Wirksamkeit von Magnetfeldern nur in den unmittelbar ausgesetzten Bereichen und ihrer direkten Nachbarschaft nachweisen. Sofern von einem biegsamen Material gesprochen wird wie Gummi, weichem Polyvinyl und Acetylcellulose, welche mit dauermagnetischem feinem Pulver gemischt sind und die angeblich streifenartig oder karoförmig magnetisiert. sein sollen, so handelt es sich mit Sicherheit nicht um ausreichend starke und deshalb therapeutisch wirksame Magnetfelder.

Die vorliegende Erfindung hat sich einerseits die Aufgabe gestellt, die bekannte magnetische Heilmethode zu verbessern, und dabei die Erfolgsquote zu erhöhen und die Handhabung zu vereinfachen, andererseits neue Anwendungen der Magnettherapie zu entwickeln.

Diese Aufgabe wird überraschenderweise gelöst durch ein Magnetpflaster, bestehend aus einer flexiblen, hautverträglichen, permanent magnetisierbaren und magnetisierten Kunststoffolie, welche selbstklebend oder mit Hilfe von hautverträglichem, selbstklebendem Pflaster auf der Haut befestigbar ist, dadurch gekennzeichnet, dass die Kunststoffolie 0,2 bis 2 mm stark, abwechselnd positiv und negativ magnetisiert ist und die positiven und negativen Pole einen Abstand von 5 mm voneinander haben und eine magnetische Flussdichte von 40 bis 200 mT aufweisen.

Um an Körperstellen, die erfahrungsgemäss stark transpirieren, eine zu starke Schweissbildung zu verhindern und das Entweichen der Feuchtigkeit zu ermöglichen, ist es empfehlenswert, das erfindungsgemässe Pflaster zu perforieren oder zu schlitzen. Erfahrungsgemäss reichen für eine derartige Perforierung Löcher mit einem Durchmesser von 1 bis 3 mm aus, die in Abständen von 3 bis 40 mm angeordnet sind. Prinzipiell können aber auch Abstände von 2 bis 100 mm, und Durchmesser von 1 bis 50 mm gewählt werden. Die Schlitze sollten 8 bis 40 mm lang, 8 bis 40 mm voneinander entfernt sein und vorzugsweise gegeneinander versetzt angeordnet sein. Die Schlitze können aber auch 2 bis 100 mm lang und in Abständen von 2 bis 100 mm vorliegen.

Die erfindungsgemässen Magnetpflaster können beliebige Formen haben, vorzugsweise sind sie rund, oval, quadratisch oder rechteckig. Bei den quadratischen und rechteckigen Formen kann es empfehlenswert sein, die Ecken etwas abzurunden, um Hautreizungen zu vermeiden. Die erfindungsgemässen Magnetpflaster sind von der Grösse her nicht beschränkt, jedoch sollten Länge und Breite im Bereich von 5 bis 1000 mm, vorzugsweise 5 bis 250 mm, liegen. Kleinere Pflaster als solche mit 5 mm Durchmesser weisen gegenüber herkömmlichen magnetischen Heilpflastern mit Ferritplättchen kaum noch Vorteile auf. Grössere Abmessungen können Schwierigkeiten bei der Anbringung auf den zu behandelnden Körperteilen bewirken, Eine bevorzugte Ausführungsform des erfindungsgemässen Magnetpflasters ist rechteckig mit abgerundeten Ecken und hat die Dimensionen 60 x 100 mm. Für gewisse Anwendungen werden streifenförmige Pflaster bevorzugt.

Prinzipiell ist es jedoch auch möglich, die erfindungsgemässen Magnetpflaster in grösseren Stücken zuzuschneiden und sie dazu zu verwenden, den Patienten hierin für eine begrenzte Zeit einzurollen. Hierzu wird es u.U. sinnvoll sein, die Magnetpflaster vorher aufzuwärmen, um die Wärmeerzeugung durch die Magnetfelder durch äusserlich zugeführte Wärme zu verstärken.

Als flexible, hautverträgliche, permanent magnetisierbare und magnetisierte Kunststoffolie für das erfindungsgemässe Magnetpflaster können alle ausreichend flexiblen, inerten und vor allem hautverträglichen Kunststoffolien verwendet werden, welche magnetisierbare Partikel, z.B. aus Ferrit oder Chromdioxid enthalten. Besonders bevorzugt sind gummielastische Kunststoffolien aus natürlichem oder synthetischem Gummi sowie Siliconkautschuk. Diese Folien werden mit Hilfe stärker Magnetfelder permanent magnetisiert. Hierbei ist es völlig ausreichend, wenn nur an der der Haut zugewandten Seite der Kunststoffolie Magnetpole entstehen. Es ist zwar prinzipiell möglich, so permanent zu magnetisieren, dass an der Haut zugewandten Oberfläche nur positive oder nur negative Pole entstehen (unipolare Pflaster mit nur Nordpolen oder Südpolen); erfindungsgemäss benutzt werden jedoch nur solche Magnetfolien, bei denen abwechselnd positive und negative Pole angeordnet sind. Diese Pole sind in Abständen von 5 mm auf der der Haut zugewandten Seite der Folie vorhanden.

Die abwechselnde Anbringung von positiven und negativen Polen hat den besonderen Vorteil, dass dadurch nicht nur grössere Flächen des Körpers der magnetischen Heilwirkung ausgesetzt werden, sondern darüberhinaus der für die betreffende Therapie optimale Pol in die Nähe des optimalen Punktes gebracht wird. Es hat sich nämlich inzwischen herausgestellt, dass gewisse Krankheitsbilder stärker auf positive und andere stärker auf negative Pole ansprechen. Bei der Therapie mit diesen erfindungsgemässen Magnetpflastern mit beiden Polarten auf der Oberfläche ist es möglich, beide Polarten zum Einsatz zu bringen und dadurch die Erfolgschancen zu erhöhen. Weiterhin dringen die Magnetfelder solcher Magnetpflaster tiefer in den Organismus ein, da es sich um gekreuzte Magnetfelder handelt.

Bei der Therapie mit den erfindungsgemässen Magnetpflastern werden insbesondere bei flächenartigen Schmerzen und Erkrankungen spürbare und messbare Wärmefelder erzeugt, die schon nach wenigen Tagen zu bemerkenswerten Heilungserfolgen führen. Patienten, bei denen die Therapie mit bisher bekannten magnetischen Heilpflastern mit Ferritplättchen keine oder nur geringe Erfolge zeigten, beobachteten mit dem erfindungsgemässen Magnetpflaster bereits nach wenigen Tagen beachtliche Linderung oder Heilung. Als flexible, hautverträgliche, permanent magnetisierbare Kunststoffolien kommen beispielsweise die Gummifolien infrage, die als selbsthaftende Schablonen bei der Beschriftung und Lackierung von Autos zur Anwendung kommen. Diese Folien werden in Stärken von 0,5, 0,9, 1 und 1,5 mm geliefert. Für die erfindungsgemässen Magnetpflaster haben sich die Folien mit einer Stärke von 0,5 mm bewährt, bei denen Feldstärken von ca. 40 mT (400 Gauss) gemessen wurden und bei der positive und negative Pole abwechselnd in streifenförmigen Abständen von 5 mm vorhanden sind. Erfindungsgemäss verwendet werden Magnetfolien mit 40 bis 200 mT (400 bis 2000 Gauss). Vorzugsweise werden jedoch speziell für die therapeutische Anwendung angepasste Folien hergestellt und verwendet.

Zur Befestigung dieser Folien auf der Haut ist entweder möglich, diese einseitig mit einem hautverträglichen Kleber zu beschichten oder sie mit Hilfe von hautverträglichen selbstklebenden Pflastern auf der Haut zu befestigen. Hautverträgliche Kleber sind dem Fachmann bekannt und werden beispielsweise bei der Herstellung herkömmlicher Pflaster verwendet. Sofern die erfindungsgemässen Magnetpflaster selbstklebend sind, ist ein enger Kontakt der Folie mit der Haut gewährleistet. An Körperstellen, die erfahrungsgemäss stark transpirieren, kann dies auch bei Perforierung oder Schlitzung der Folie zu gewissen Schwierigkeiten führen. Insbesondere wenn Folien verwendet werden, die gegen Lösungsmittel empfindlich sind, die für die Aufbringung hautverträglicher Kleber verwendet werden, ist es durchaus möglich, Magnetfolien mit Hilfe selbstklebender Pflaster auf der Haut zu befestigen. Im Prinzip reicht es dabei, wenn dieses selbstklebende Pflaster zum Teil an der Magnetfolie und zum Teil an der Haut klebt. Da die Magnetfolien im allgemeinen jedoch dunkel gefärbt sind, kann es vorteilhaft sein, die gesamte Magnetfolie mit einem selbstklebenden Pflaster abzudecken und sie mit einem überstehenden Streifen des selbstklebenden Pflasters an der Haut zu befestigen. Dadurch, dass man selbstklebende Pflaster leicht hautfarben einfärben kann, ist die Befestigung des erfindungsgemässen Magnetpflasters optisch weniger auffällig, zumal eine dunkle Folie durch dünnere Wäschestücke hindurch sichtbar bleibt.

Bei erfindungsgemässen Magnetpflastern, die selbstklebend sind, empfiehlt es sich, die klebende Schicht durch siliconiertes Papier abzudecken Diese Papierschicht kann dann vor der Verwendung am Körper abgezogen und verworfen werden.

Es ist prinzipiell möglich, die Rückseite dieser selbstklebenden Magnetpflaster hautfarben mit einer elastischen Textilschicht zu kaschieren um damit das Pflaster optisch weniger auffällig zu gestalten. Weiterhin besteht die Möglichkeit die Magnetfolie auf der von der Haut abweisenden Seite hell oder metallisch zu beschichten bzw anzufärben. Metallische Beschichtungen haben obendrein eine reflektierende und somit verstärkende Wirkung.

Vergleichende Untersuchungen an Gesunden über die Hauttemperatur und ihre Veränderung bei Einwirkung der erfindungsgemässen Magnetpflaster und herkömmlichen Pflastern mit durchblutungsfördernden Inhaltsstoffen, beispielsweise auf Basis von Extractum et Fructus Capsici, haben ergeben, dass bei den erfindungsgemässen Magnetpflastern keine messbaren Temperatursteigerungen entstehen, während bei herkömmlichen Heilpflastern mit durchblutungsfördernden Pflanzenextrakten Temperatursteigerungen von 3 °C unterhalb des Pflasters und 1 bis 2°C in einem Abstand bis zu 10 cm vom Pflaster feststellbar sind. Die bei Anwendung der erfindungsgemässen Heilpflaster verspürte Wärmesteigerung ist somit subjektiv und beruht nur auf einer Veränderung der Stoffwechsellage in den Zellen und dem umliegenden Bindegewebe, während bei herkömmlichen Pflastern mit Wirkstoffen eine gewisse künstliche Entzündung durch höhere Durchblutung der Haut erzeugt wird.

Da es sich somit um zwei völlig verschiedene Wirkprinzipien handelt, ist es prinzipiell möglich, diese miteinander zu kombinieren. Dazu wird in einer bevorzugten Ausführungsform der erfindungsgemässen Magnetpflaster eine selbstklebende Schicht gewählt, die zusätzlich eine durchblutungsfördernde Substanz enthält. Als durchblutungsfördernde Substanzen kommen alle bereits bekannten und in der Therapie bewährten Substanzen in Frage. Besonders bevorzugt sind durchblutungsfördernde Substanzen aus Senf, Paprica oder Fructus Capsici. Ein Gemisch aus Extractum et Fructus Capsici, entsprechend etwa 0,05 bis 5%, vorzugsweise 0,1 bis 3%, hat sich bei herkömmlichen Heilpflastern bewährt und ist deshalb auch prinzipiell geeignet, in dieser Menge in die selbstklebende Schicht der erfindungsgemässen Magnetpflaster eingebracht zu werden.

Da erfindungsgemässe Magnetpflaster mit dem Zusatz durchblutungsfördernder Substanzen im doppelten Sinne wirksam sind, ist auch eine raschere und intensivere Wirkung die Folge.

Messungen an unterkühltem, schlecht durchblutetem Gewebe ergaben, dass auch erfindungsgemässe Magnetpflaster ohne durchblutungsfördernde Substanzen messbare Temperaturerhöhungen bewirken.

In den nachfolgenden beispielen sind einige Ausführungsformen der erfindungsgemässen Magnetpflaster näher erläutert. Es ist jedoch für den Fachmann selbstverständlich, dass auch andere Ausführungsformen mit anderen Grössen und anderen Materialien zu entsprechenden Ergebnissen führen werden.

Beispiel 1

Eine 0,5 mm starke Magnet-Gummifolie (Lieferant Serimag, Leutschenbachstrasse 71, Zürich) mit den Abmessungen 125 x 100 mm wurde mit einem hautfarbenen, selbstklebenden, hautverträglichen Pflaster auf Körperteilen und Hautpartien mit starken rheumatischen Beschwerden aufgeklebt. Bereits nach kurzer Zeit wurde von den Patienten eine spürbare Erwärmung und bessere Durchblutung dieser Körperteile verspürt. Nach 2 bis 5 Tagen wurde das Pflaster entfernt. In einigen Fällen wurde nur eine erhebliche Linderung. in anderen Fällen völliges Verschwinden der Schmerzen beobachtet. In den Fällen, in denen nur Linderung eingetreten war, wurde nach 1 bis 2 Tagen die Therapie wiederholt, wobei eine weitere Linderung beobachtet wurde.

Beispiel 2

Eine Magnetfolie gemäss Beispiel 1 wurde im Abstand vor 10 mm mit 2 mm grossen Löchern versehen (perforiert) und danach in entsprechender Weise mit hautfarbenem, selbstklebendem, hautverträglichem Pflaster befestigt. Die unter der Folie befindliche Hautpartie war nach der Therapie weniger durch Schweiss beeinflusst. Die Heilerfolge waren jedoch nicht beeinträchtigt.

Beispiel 3

Eine Magnetfolie gemäss Beispiel 1 wurde im Abstand von 10 mm mit 35 mm langen Schlitzen versehen, die gegeneinander versetzt angeordnet sind. Die Magnetfolie war einseitig mit einem hautverträglichen Kleber überzogen und mit silikoniertem Papier abgedeckt. Das silikonierte Papier liess sich vor der Behandlung leicht abziehen und das Pflaster ohne Schwierigkeiten an den gewünschten Stellen des Körpers aufkleben. Die Schlitze erhöhten die Elastizität des Pflasters und gestatteten gleichzeitig eine bessere Transpiration. Die Heilerfolge waren die gleichen wie im Beispiel 1 beobachtet.

Eine Untersuchung der Magnetfolie der Firma Serimag ergab, dass sie abwechselnd Plus- und Minus-Pole streifenförmig in einem Abstand aufwies. Die Feldstärken betrugen abwechselnd plus und minus 40 mT (400 Gauss). Die Folien sind hautverträglich, gummielastisch und so flexibel, dass sie sich ohne Schwierigkeiten an die Körperteile anschmiegen, insbesondere wenn sie obendrein geschlitzt sind.

Beispiel 4

Eine Magnetfolie gemäss Beispiel 1 mit den Abmessungen 60 x 100 mm wurde im Abstand von 15 mm mit versetzten 15 mm langen Schlitzen versehen. Die Magnetfolie war einseitig mit einem hautverträglichen Kleber überzogen, welcher 3% Extractum et Fructus Capsici enthielt. Die Klebeschicht wurde mit siliconiertem Papier abgedeckt. Das siliconierte Papier liess sich vor der Behandlung leicht abziehen und das Pflaster ohne Schwierigkeiten an gewünschten Stellen des Körpers aufkleben. Die Schlitze erhöhen die Elastizität des Pflasters und gestatten gleichzeitig eine bessere Transpiration. Der Zusatz der durchblutungsfördernden Substanz führt zu subjektiv und objektiv stärkerer Erwärmung der abgedeckten Teile, die mit stärkeren und rascheren Heilerfolgen verbunden ist.

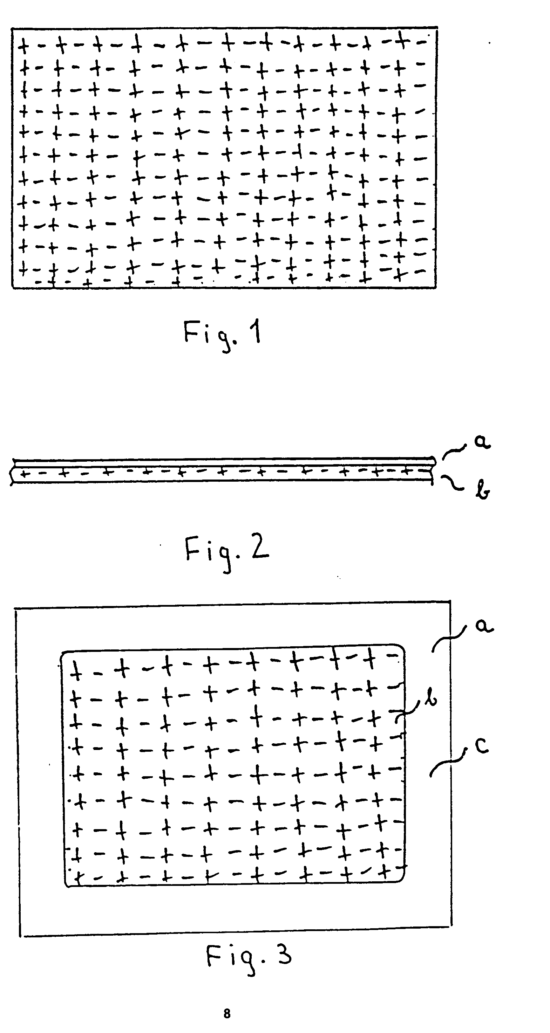

In den Figuren 1 bis 3 sind typische Ausführungsformen der erfindungsgemässen Magnetpflaster dargestellt. Figur 1 zeigt in der Aufsicht eine selbstklebende Magnet-Gummifolie mit abwechselnden Streifen von (+)-Polen und (-)-Polen. Figur 2 zeigt die gleiche Folie wie Figur 1 im Schnitt, wobei a die selbstklebende Schicht und b die Magnetfolie darstellt. Figur 3 zeigt eine Ausführungsform, bei der die Magnetfolie vollständig von einem selbstklebenden Pflaster abgedeckt ist und bei der a die selbstklebende Schicht des Pflasters, b die Magnetfolie und c die Pflasterschicht darstellt, die vorzugsweise hautfarben und aus Gewebe oder elastischem Plastikmaterial besteht. Die dargestellten Magnetpflaster können zusätzlich in der oben beschriebenen Weise perforiert oder geschlitzt werden.

**Patentansprüche**

1.  Magnetpflaster bestehend aus einer flexiblen, hautverträglichen, permanent magnetisierbaren und magnetisierten Kunststoffolie (b), welche selbstklebend oder mit Hilfe von hautverträglichem, selbstklebendem Pflaster (C) auf der Haut befestigbar ist, dadurch gekennzeichnet, dass die Kunststoffolie (b) 0,2 bis 2 mm stark, abwechselnd positiv und negativ magnetisiert ist und die positiven und negativen Pole einen Abstand von 5 mm voneinander haben und eine magnetische Flussdichte von 40 bis 200 mT aufweisen.

2.  Magnetpflaster gemäss Anspruch 1, dadurch gekennzeichnet, dass die selbstklebende Schicht zusätzlich eine durchblutungsfördernde Substanz enthält.

3.  Magnetpflaster gemäss Anspruch 2 dadurch gekennzeichnet, dass die durchblutungsfördernde Substanz aus Senf, Paprica oder Fructus Capsici gewonnen wurde und in einer Menge von 0,05 bis 5 Gew.-% der selbstklebenden Schicht vorliegt

4.  Magnetpflaster gemäss einem der Ansprüche 1 bis 3, dadurch gekennzeichnet, dass es perforiert oder geschlitzt ist.

5.  Magnetpflaster nach einem der Ansprüche 1 bis 4, dadurch gekennzeichnet, dass es rund, oval, quadratisch oder rechteckig ist und die Länge und Breite 5 bis 1000 mm betragen.

6.  Magnetpflaster nach einem der Ansprüche 1 bis 5, dadurch gekennzeichnet, dass die Magnetpole als abwechselnde parallele Streifen vorliegen.

7.  Magnetpflaster gemäss einem der Ansprüche 1 bis 6, dadurch gekennzeichnet, dass die Schlitze in Abständen von 2 bis 100 mm vorliegen, 2 bis 100 mm lang sind und gegeneinander versetzt angeordnet sind.

8.  Magnetpflaster gemäss einem der Ansprüche 1 bis 6, dadurch gekennzeichnet, dass die Löcher der Perforation in Abständen von 2 bis 100 mm vorliegen und 1 bis 50 mm Durchmesser aufweisen.

9.  Magnetpflaster gemäss einem der Ansprüche 1 bis 8, dadurch gekennzeichnet, dass die Kunststoffolie aus gummielastischem Material besteht.

10. Verwendung von 0,2 bis 5 mm starken, flexiblen, hautverträglichen permanent magnetisierten Kunststoffolien, die abwechselnd positiv und negativ magnetisiert sind und bei denen die positiven und negativen Pole einen Abstand von 5 mm voneinander haben und eine magnetische Flussdichte von 40 bis 200 mT aufweisen, zur Herstellung von selbstklebenden oder mit Hilfe von hautverträglichem selbstklebendem Pflaster auf der Haut befestigbaren Magnetpflastern.

**Claims**

1.  A magnetic plaster consisting of a flexible, permanently magnetizable and magnetized plastic sheet (b) which is compatible with the skin and is self-adhesive or is attachable to the skin by means of a self-adhesive plaster (C) compatible with the skin, characterized in that the plastic sheet has a thickness of from 0.2 to 2 mm, it has alternatingly been positively and negatively magnetized, and the positive and negative poles are at distances between each other of 5 mm and exhibit a magnetic flux density of from 40 to 200 mT.

2. The magnetic plaster according to claim 1, characterized in that the self-adhesive layer additionally contains a substance which stimulates the circulation of the blood.

3. The magnetic plaster according to claim 2, characterized in that the substance which stimulates the circulation of the blood has been recovered from mustard, paprica or Fructus Capsici and is present in an amount of from 0.05 to 5 per cent by weight of the self-adhesive layer.

4. The magnetic plaster according to any one of claims 1 to 3, characterized in that it has been perforated or slit.

5. The magnetic plaster according to any one of claims 1 to 4, characterized in that it has circular, oval, square or rectangular shape and its length and width are from 5 to 1,000 mm.

6. The magnetic plaster according to any one of claims 1 to 5, characterized in that the magnetic poles are present in an arrangement of alternating parallel strips.

7. The magnetic plaster according to any one of claims 1 to 6, characterized in that the slits are present at distances of from 2 to 100 mm between each other, have a length of from 2 to 100 mm each, and are arranged in staggered positions to each other.

8. The magnetic plaster according to any one of claims 1 to 6, characterized in that the holes of the perforation are present at distances of from 2 to 100 mm between each other and have a diameter of from 1 to 50 mm each.

9. The magnetic plaster according to any one of claims 1 to 8, characterized in that the plastics sheet consists of a material having rubber elasticity.

10. Use of flexible, permanently magnetized plastic sheets which are 0.2 to 5 mm in thickness and are compatible with the skin and have alternatingly been positively and negatively magnetized and wherein the positive and negative poles are at distances between each other of 5 mm and exhibit a magnetic flux density of from 40 to 200 mT for the manufacture of magnetic plasters which are self-adhesive or attachable to the skin by means of a self-adhesive plaster compatible with the skin.

## Revendications

1. Emplâtre magnétique constitué d'une feuille de matière plastique (b) flexible, compatible avec la peau, susceptible de recevoir une aimantation permanente et aimantée, susceptible d'être fixée sur la peau par auto-collage ou à l'aide d'un emplâtre (c) auto-collant et compatible avec la peau, caractérisé en ce que la feuille de matière plastique (b), d'une épaisseur de 0,2 à 2 mm, est aimantée alternativement positivement et négativement, les pôles positifs et négatifs étant espacés les uns des autres de 5 mm, et présentant une densité de flux magnétique de 40 à 200 mT.

2. Emplâtre selon la revendication 1, caractérisé en ce que la couche auto-collante contient, en outre, une substance accélérant la circulation du sang.

3. Emplâtre selon la revendication 2, caractérisé en ce que ladite substance accélérant la circulation du sang est obtenue à partir de moutarde PAPRICA ou FRUCTUS CAPSICI, et se trouve dans une proportion de 0,05 à 5% en poids de la couche auto-collante.

4. Emplâtre selon l'une des revendications 1 à 3, caractérisé en ce qu'il est perforé ou présente des fentes.

5. Emplâtre selon l'une des revendications 1 à 4, caractérisé en ce qu'il est rond, ovale, carré ou rectangulaire, avec une longueur et une largeur de 5 à 1000 mm.

6. Emplâtre selon l'une des revendications 1 à 5, caractérisé en ce que les pôles magnétiques sont disposés selon des files parallèles alternées.

7. Emplâtre selon l'une des revendications 1 à 6, caractérisé en ce que les fentes sont à des espacements de 2 à 100 mm, ont une longueur de 2 à 100 mm, et sont décalées les unes par rapport aux autres.

8. Emplâtre selon l'une des revendications 1 à 6, caractérisé en ce que les perforations sont à des espacements de 2 à 100 mm et ont un diamètre de 1 à 50 mm.

9. Emplâtre selon l'une des revendications 1 à 8, caractérisé en ce que la feuille de matière plastique est en une matière élastique analogue à du caoutchouc.

10. Application de feuilles de matière plastique flexible compatible avec la peau, d'épaisseur de 0,2 à 5 mm, susceptibles de recevoir une aimantation permanente, aimantées alternativement positivement et négativement avec des pôles positifs et négatifs espacés les uns des autres de 5 mm, et ayant un flux magnétique de 40 à 200 mT, à la réalisation d'emplâtres magnétiques qu'on peut fixer sur la peau par auto-collage ou à l'aide d'un emplâtre auto-collant compatible avec la peau.

Fig. 1

Fig. 2

Fig. 3